# EUROPEAN PATENT APPLICATION

(11) **EP 3 723 015 A1**
(43) Date of publication of application: **14.10.2020**
(21) Application number: 18901748.6
(22) Date of filing: 16.01.2018
(51) Int. Cl.: G06Q 10/10, G16H 10/20, G06Q 50/22

(54) **CLINICAL TRIAL SYSTEM FOR TREATMENT APPLICATION, TERMINAL DEVICE HAVING TREATMENT APPLICATION MOUNTED THEREON, AND TREATMENT APPLICATION PROGRAM**

(71) Applicant: Sustainable Medicine, Inc., Chuo-ku Tokyo 103-0023 (JP)
(72) Inventor: UENO, Taro, Tokyo 103-0023 (JP)
(74) Representative: Michalski Hüttermann & Partner Patentanwälte mbB
(86) International application number: PCT/JP2018/001017
(87) International publication number: WO 2019/142238

(57) **Abstract**

A clinical trial management server 300 randomly assigns a subject to either a therapeutic group or a control group on the basis of subject assignment factor information input from a doctor terminal 200, and transmits assignment information to a subject terminal 100. In addition, in the subject terminal 100 with which the subject uses a therapeutic application, the therapeutic application is set to either a first mode that provides an actual therapeutic function or a second mode that provides a pseudo-therapeutic function on the basis of the assignment information transmitted from the clinical trial management server 300. According to this, both a subject and a doctor cannot understand that the subject is assigned to either a therapeutic group or a control group and the therapeutic application operates in which mode, and in the case of performing a clinical trial that applies a placebo-controlled test with respect to the therapeutic application, it is possible to systemically realize randomization and double-blindness of assignment.

## Description

### Technical Field

The present invention relates to a clinical trial system for a therapeutic application, a terminal device equipped with a therapeutic application, and a therapeutic application program.

### Background Art

According to revision of Medical Products and Medical Devices Act (formerly Pharmaceutical Affairs Law) in the fall of 2014, medical software has been handled as medical equipment, and as a result, attempts have been made to utilize a smartphone in which a dedicated application for disease therapy (hereinafter, referred to as "therapeutic application") is installed for disease therapy. For example, development of a therapeutic application aimed at treating diseases such as nicotine addiction and sleep disturbance by improving lifestyle of patients is in progress.

In order to actually utilize the therapeutic application as a medical device, it is necessary to obtain approval pursuant to the Medical Products and Medical Devices Act. In addition, in order to obtain the approval, it is necessary to carry out a clinical test (clinical trial) for verifying effectiveness of a therapeutic effect using the therapeutic application. The clinical trial of the therapeutic application can be conducted by applying, for example, a placebo-controlled test that is typically conducted in clinical tests of drugs.

The placebo-controlled test is a test in which a plurality of subjects are divided into a therapeutic group and a control group, a drug is actually assigned to the therapeutic group, and a placebo is assigned to the control group for verifying drug efficacy. The placebo is a placebo that mimics physical characteristics such as a color, a weight, a taste, and an odor to an active drug as closely as possible, and does not contain medicinal components. Typically, the placebo-controlled test is conducted in a double-blind state in which a plurality of the subjects are randomly assigned to the active drug or the placebo, and neither a doctor nor the subjects know the assignment.

In a case where a patient uses a drug and symptoms improve, the cause includes not only a therapeutic effect of the drug itself, but also various factors such as natural course of the disease, an effect due to participation in the test, and subjective factors of diagnosis and evaluation. In contrast, when conducting the placebo-controlled test under randomization and double-blindness, all potential effects other than investigational drug action can be controlled.

In the related art, there is known a clinical test execution management system capable of conducting total management of the placebo-controlled test (for example, refer to Patent Document 1). According to the clinical test execution management system described in Patent Document 1, automatic assignment of subjects is executed in accordance with factors including an age, a sex, a complication, and a medical history so that an active drug target and a placebo target are in a distributed state.

That is, a clinical test institution name, a subject name, a subject identification code, a clinical test attending physician, a clinical test chief physician name, an age, a sex, a complication, a medical history, confirmation of consent, and the like are input to subjects who pass a subject profile filter, and the subjects are registered in a state of being randomly assigned to either an active drug target or a placebo target so that a target distribution is in an appropriate distributed state.

In addition, recently, information relating to clinical trials of the therapeutic application for the nicotine addiction has been published (for example, refer to Non-Patent Document 1). Non-Patent Document 1 discloses that with regard to verification of effectiveness of the therapeutic application, a clinical trial that applies a placebo-controlled test of a drug will be conducted. That is, a plurality of patients diagnosed as nicotine addiction in a screening test are randomly assigned to either a clinical trial therapeutic group or a control group. In addition, in addition to a standard therapeutic program, a therapeutic application is introduced to the clinical trial therapeutic group and a sham (pseudo-therapy) application is introduced to the control group for therapy. Specifically, after 12 weeks for smoking cessation outpatient therapy, a patient is caused to use either the therapeutic application or the sham application until 24 weeks to evaluate a continuous smoking cessation rate from 9 weeks to 24 weeks.

Patent Document 1: JP-A-2006-323864

Non-Patent Document 1: The future where an application "treats" is approaching! Japanese first "smartphone application clinical trial", starting with nicotine addiction therapy, 2017.10.23, http://healthcare-biz.jp/2017/10/%E3%82%A2%E3%83%97%E3%83%AA%E3%81 %8C%E6%B2%BB%E7%99%82%E3%81%99%E3%82%8B%E6%9C%AA%E6%9 D%A5%E3%81%8C%E8%BF%91%E3%81%A5%E3%81%84%E3%81%9F%EF%BC%81-%E6%97%A5%E6%9C%AC%E5%88%9D%E3%80%8C/

### Disclosure of the Invention

Clinical trial of the therapeutic application is not conducted so far, and in the case of conducting the clinical trial that applies the placebo-controlled test, it is important how to realize randomization and double-blindness. Non-Patent Document 1 includes description relating to the randomization but does not mention how to realize the double-blindness.

On the other hand, Patent Document 1 includes description relating to blindness other than the randomization. With regard to the randomization, in a system described in Patent Document 1, a person in charge of clinical test institution accesses a data center by using a wide area information transmission tool, and a drug number is applied by random assignment relating to medication on a database of the clinical test of the data center. However, with regard to blinding, only "this system naturally satisfies the criteria of blindness" is described, and how to specifically realize the blindness is not described.

The invention has been made to solve such a problem, and an object thereof is to provide a method for systematically realizing randomization and double-blindness of assignment to a therapeutic group and a control group in the case of conducting a clinical trial that applies a placebo-controlled test for a therapeutic application.

To accomplish the object, according to an aspect of the invention, there is provided a clinical trial system for a therapeutic application. In a clinical trial management server that performs management of a clinical trial, a subject is randomly assigned to either a therapeutic group or a control group so that a target distribution is in an appropriate distributed state on the basis of subject assignment factor information input in combination with subject specifying information from a doctor terminal, and assignment information indicating a result of assignment to either the therapeutic group or the control group is transmitted to a subject terminal. In addition, in the subject terminal with which the subject uses the therapeutic application, the therapeutic application is set to either a first mode that provides an actual therapeutic function or a second mode that provides a pseudo-therapeutic function on the basis of the assignment information transmitted from the clinical trial management server.

According to the invention having the above-described configuration, in the clinical trial management server, assignment of the subject to either the therapeutic group or the control group is randomly performed on the basis of the subject assignment factor information, and thus randomization of assignment to the therapeutic group and the control group is realized. In addition, a result of the assignment is not disclosed to either the doctor terminal or the subject terminal, and the therapeutic application is set to either the first mode that provides an actual therapeutic function or the second mode that provides a pseudo-therapeutic function on the basis of the assignment information transmitted from the clinical trial management server to the subject terminal. Accordingly, both the subject and a doctor cannot understand that the subject is assigned to either the therapeutic group or the control group, and the therapeutic application operates in which mode. According to this, in the case of performing a clinical trial that applies a placebo-controlled test with respect to the therapeutic application, it is possible to systemically realize randomization and double-blindness of assignment to a therapeutic group and a control group.

### Brief Description of the Drawings

Fig. 1 is a diagram illustrating a network configuration of a clinical trial system for a therapeutic application according to first and second embodiments.
Fig. 2 is a block diagram illustrating a functional configuration example of a subject terminal and a clinical trial management server according to the first embodiment.
Fig. 3 is a diagram illustrating an example of one record of an assignment database stored in an assignment database storage unit according to the first embodiment.
Fig. 4 is a flowchart illustrating an operation example of the clinical trial system according to the first embodiment.
Fig. 5 is a block diagram illustrating a functional configuration example of a subject terminal and a clinical trial management server according to the second embodiment.
Fig. 6 is a diagram illustrating an example of one record of an assignment database stored in an assignment database storage unit according to the second embodiment.
Fig. 7 is a flowchart illustrating an operation example of the clinical trial system according to the second embodiment.
Fig. 8 is a diagram illustrating a network configuration of a clinical trial system for a therapeutic application according to a third embodiment.
Fig. 9 is a block diagram illustrating a functional configuration example of a subject terminal and a clinical trial management server according to the third embodiment.
Fig. 10 is a diagram illustrating an example of one record of each of an assignment database stored in an assignment database storage unit and an authentication database stored in an authentication database storage unit according to the third embodiment.
Fig. 11 is a flowchart illustrating an operation example of the clinical trial system according to the third embodiment.

### Best Mode for Carrying Out the Invention

### (First Embodiment)

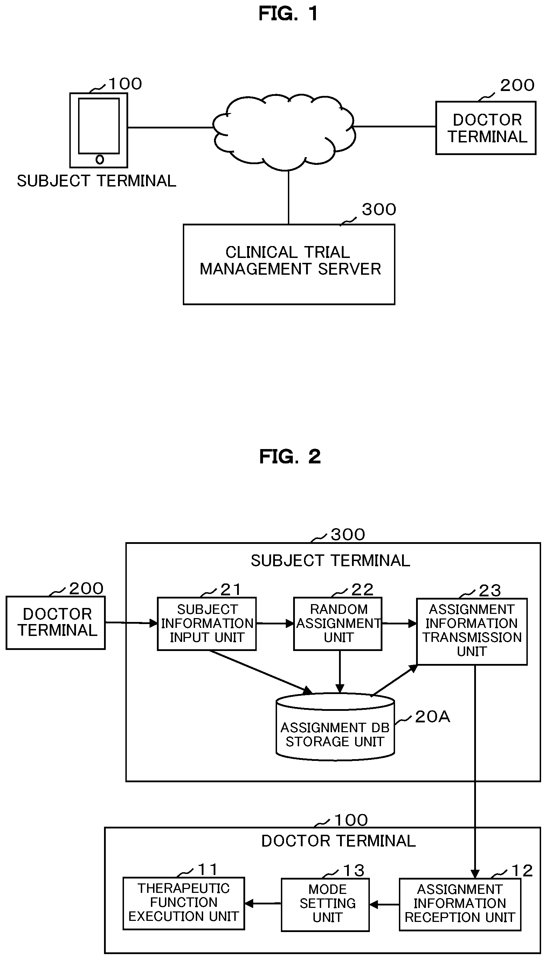

Hereinafter, a first embodiment of the invention will be described with reference to the accompanying drawings. Fig. 1 is a diagram illustrating a network configuration of a clinical trial system for a therapeutic application according to the first embodiment.

As illustrated in Fig. 1, the clinical trial system for a therapeutic application according to the first embodiment (hereinafter, simply referred to as "clinical trial system") is a system including a subject terminal 100 as a terminal device with which a subject uses a therapeutic application, a doctor terminal 200 that is used by a doctor, and a clinical trial management server 300 that performs management of a clinical trial. The subject terminal 100, the doctor terminal 200, and the clinical trial management server 300 conduct the clinical trial of the therapeutic application in cooperation.

The therapeutic application is an application for therapy through improvement of a lifestyle of a subject by inputting information relating to a symptom, a daily behavior, or the like of a subject at any time, performing a predetermined analysis on the basis of the input information, and outputting a message relating to predetermined advice or executing a predetermined process accompanied with a behavior of the subject. Note that, the analysis may be performed by the therapeutic application itself, or may be performed by the clinical trial management server 300 or another analysis-dedicated server.

In this embodiment, the therapeutic application includes a program (hereinafter, referred to as "actual therapeutic program") coded for providing an actual therapeutic function, and a program (hereinafter, referred to as "pseudo-therapeutic program") coded for providing a pseudo-therapeutic function. The actual therapeutic function represents a function for carrying out the content for which a therapeutic effect through improvement of the lifestyle of the subject is actually expected with respect to the predetermined advice or the predetermined process. On the other hand, the pseudo-therapeutic function represents a function for carrying out the content for which the therapeutic effect through improvement of the lifestyle of the subject is not expected with respect to the predetermined advice or the predetermined process.

The subject uses the therapeutic application in a state in which either the actual therapeutic program or the pseudo-therapeutic program is executed (a state in which either the following first mode or the following second mode is set). However, it is not known that a program of which function is being executed. That is, the clinical trial system of this embodiment performs a clinical trial of the therapeutic application by applying a placebo-controlled test that is typically performed in a drug clinical test. Note that, a subject who uses the therapeutic application in a state in which the actual therapeutic program is executed pertains to a therapeutic group, and a subject who uses the therapeutic application in a state in which the pseudo-therapeutic program is executed pertains to a control group.

The subject terminal 100 that is used by the subject may be any device as long as the therapeutic application can be installed and executed. For example, a smartphone, a tablet, a PC, or the like can be used as the subject terminal 100. However, the therapeutic application is aimed at therapy through improvement of a lifestyle of the subject, and thus it is preferable to use a mobile-type terminal capable of being carried at all times.

The doctor terminal 200 that is used by a doctor is a terminal in which a therapeutic application for doctors, which operates in conjunction with the therapeutic application for subjects which is installed in the subject terminal 100, is installed. Any of a smartphone, a tablet, a PC, and the like can be applied to the terminal as long as the application can be installed and executed. Note that, in a case where "therapeutic application" is simply noted in the following description, this represents the application for subjects unless otherwise stated.

The clinical trial management server 300 is a management device in which a dedicated application operating in conjunction with respective applications installed in the subject terminal 100 and the doctor terminal 200 is installed. Particularly, in this embodiment, the clinical trial management server 300 executes a process of randomly assigning a plurality of subjects to either the therapeutic group or the control group, and a process necessary for a determination for causing the therapeutic application to operate as one providing either the actual therapeutic function or the pseudo-therapeutic function. Details thereof will be described later.

Fig. 2 is a block diagram illustrating a functional configuration example of the subject terminal 100 and the clinical trial management server 300 according to the first embodiment. As illustrated in Fig. 2, the subject terminal 100 according to the first embodiment includes a therapeutic function execution unit 11, an assignment information reception unit 12, and a mode setting unit 13 as a functional configuration.

The respective functional blocks 11 to 13 are realized by execution of the therapeutic application installed in the subject terminal 100. In this case, actually, the respective functional blocks 11 to 13 include a CPU, a RAM, a ROM, and the like of a computer, and are realized when a therapeutic application program stored in a recording medium such as the RAM, the ROM, a hard disk, and a semiconductor memory operates.

Note that, as described above, the therapeutic application includes the actual therapeutic program and the pseudo-therapeutic program. The therapeutic function execution unit 11 is realized when either the actual therapeutic program or the pseudo-therapeutic program operates. On the other hand, the assignment information reception unit 12 and the mode setting unit 13 are realized when a basic program different from the actual therapeutic program and the pseudo-therapeutic program operates.

The clinical trial management server 300 according to the first embodiment includes a subject information input unit 21, a random assignment unit 22, and an assignment information transmission unit 23 as a functional configuration. In addition, the clinical trial management server 300 includes an assignment database storage unit 20A as a storage medium.

The respective functional blocks 21 to 23 can be constituted by any of hardware, a digital signal processor (DSP), and software. For example, in the case of being constituted by the software, actually, the respective functional blocks 21 to 23 include a CPU, a RAM, a ROM, and the like of a computer, and are realized when a program stored in a recording medium such as the RAM, the ROM, a hard disk, and a semiconductor memory operates.

The subject information input unit 21 of the clinical trial management server 300 inputs subject specifying information capable of specifying a subject, and assignment factor information that is referred to when assigning the subject to either the therapeutic group or the control group from the doctor terminal 200, and registers the input information to an assignment database of the assignment database storage unit 20A. The subject specifying information is information that includes a name, a sex, an age, an address, a symptom, and the like of a subject, and can specify an individual subject. The assignment factor information is information necessary when assigning the subject to either the therapeutic group or the control group so that a target distribution is in an appropriate distributed state, and as the assignment factor information, for example, information indicating the severity of symptoms is used.

Inputting of the subject specifying information and the assignment factor information is performed by a doctor with respect to the doctor terminal 200 through diagnosis on a patient. That is, when the doctor determines that the patient is to be registered as a clinical trial target subject through diagnosis, access is made from the doctor terminal 200 to the clinical trial management server 300, and the subject specifying information and the assignment factor information input to the doctor terminal 200 are transmitted to the clinical trial management server 300. In correspondence with this, the subject information input unit 21 of the clinical trial management server 300 inputs the subject specifying information and the assignment factor information transmitted from the doctor terminal 200. When the subject specifying information and the assignment factor information are input, the subject information input unit 21 stores the information in the assignment database storage unit 20A as a piece of information of the assignment database.

A patient registered as a subject accesses a predetermined download site given in notification from a doctor, and downloads the therapeutic application and installs the therapeutic application in the subject terminal 100.

The random assignment unit 22 randomly assigns the subject to either the therapeutic group or the control group so that a target distribution is in an appropriate distributed state on the basis of the assignment factor information input by the subject information input unit 21, and registers assignment information indicating the assignment result in the assignment database. Here, after registration of the required number of subjects (storage of the subject specifying information and the assignment factor information in the assignment database) is completed, the random assignment unit 22 executes a process of randomly assigning respective subjects to either the therapeutic group or the control group. The random assignment unit 22 stores assignment information indicating the assignment result in the assignment database storage unit 20A in association with the subject specifying information in the assignment database.

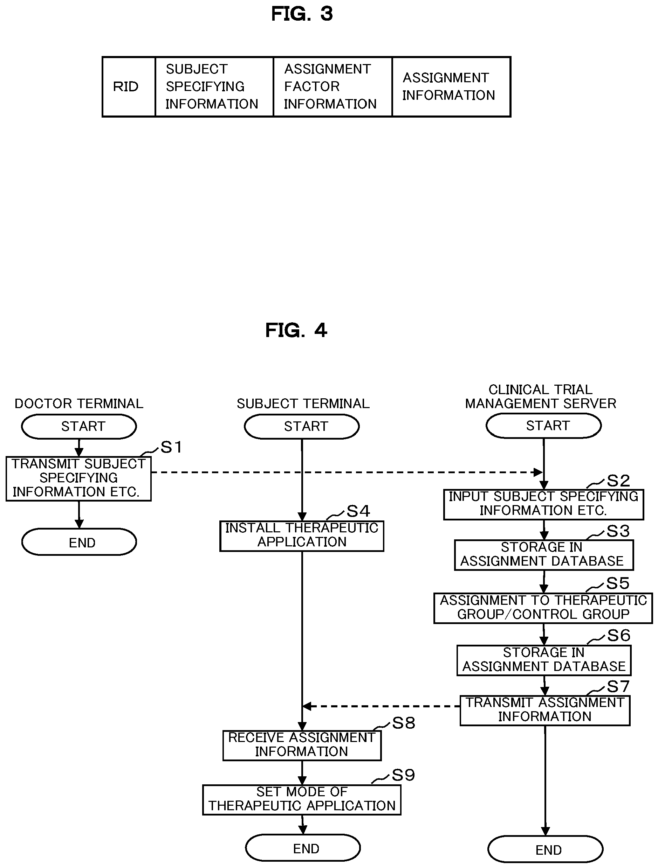

Fig. 3 is a diagram illustrating an example of one record of the assignment database stored in the assignment database storage unit 20A. Information relating to one subject is recorded in one record. As illustrated in Fig. 3, the one record of the assignment database includes a record ID (RID), the subject specifying information, the assignment factor information, and the assignment information. In a stage in which the subject information input unit 21 inputs the subject specifying information and the assignment factor information, these pieces of information are recorded in the record, and in a state in which assignment is performed by the random assignment unit 22, the assignment information is additionally recorded in the record. Note that, the pieces of information noted here are pieces of minimum information necessary for describing the first embodiment, and may include other pieces of information.

The assignment information transmission unit 23 reads out the assignment information of each subject from the assignment database storage unit 20A and transmits the assignment information to the subject terminal 100. Transmission of the assignment information is implicitly performed in an aspect in which only the therapeutic application is recognizable in a state in which the transmission is not disclosed to the subject. That is, the assignment information transmission unit 23 implicitly transmits the assignment information to the therapeutic application installed in the subject terminal 100. The therapeutic application uses the received assignment information in a setting mode to be described later, but does not disclose the assignment information in any form such as displaying on a display of the subject terminal 100 and audio outputting from a speaker.

Note that, for example, destination information of the subject terminal 100 which is used when transmitting the assignment information to the subject terminal 100 is transmitted from the doctor terminal 200 to the clinical trial management server 300, and is registered therein as a piece of subject specifying information. In this case, the assignment information transmission unit 23 transmits the assignment information to the therapeutic application of the subject terminal 100 in a push type.

As another example of a method of transmitting the assignment information, the assignment information may be transmitted in a pull type. That is, access may be made to the clinical trial management server 300 from the subject terminal 100 in which installation of the therapeutic application is completed to make a request for the assignment information, and as a response for the request, the assignment information transmission unit 23 may transmit the assignment information to the subject terminal 100. In this case, address information used in access to the clinical trial management server 300 is retained by the therapeutic application in advance.

As still another example, the assignment information transmission unit 23 may indirectly transmit the assignment information to the subject terminal 100 through the doctor terminal 200. That is, the assignment information transmission unit 23 transmits the assignment information to the therapeutic application for doctors which is installed in the doctor terminal 200 in a push type or a pull type. The therapeutic application for doctors which has received the assignment information is automatically transmits the received assignment information to the subject terminal 100.

Destination information of the subject terminal 100 in this case is given in notification from a subject, and is registered in the doctor terminal 200 in advance. Alternatively, when the assignment information transmission unit 23 transmits the assignment information to the doctor terminal 200, destination information of the subject terminal 100 which is stored in advance in the assignment database storage unit 20A as one piece of the subject specifying information may be transmitted in combination.

The therapeutic function execution unit 11 of the subject terminal 100 executes a therapeutic function of the therapeutic application. That is, as described above, the therapeutic function execution unit 11 inputs information relating to a symptom, a daily behavior, or the like of a subject at any time, performs predetermined analysis on the basis of the input information, and outputs a message relating to predetermined advice or executes a predetermined process accompanied with a behavior of the subject. At this time, in the therapeutic function execution unit 11, the therapeutic application independently executes the process, or the therapeutic application executes the process in conjunction with the clinical trial management server 300 or another analysis-dedicated server.

As described above, the therapeutic application includes an actual therapeutic program or a pseudo-therapeutic program. In accordance with a situation in which the subject is assigned to either the therapeutic group or the control group in the clinical trial management server 300, the therapeutic function execution unit 11 executes various processes on the basis of either the actual therapeutic program or the pseudo-therapeutic program.

The assignment information reception unit 12 receives the assignment information transmitted from the clinical trial management server 300. The mode setting unit 13 sets the therapeutic application to either a first mode that provides an actual therapeutic function or a second mode that provides a pseudo-therapeutic function on the basis of the assignment information received by the assignment information reception unit 12.

In a case where the first mode is set by the mode setting unit 13, the therapeutic function execution unit 11 executes various processes on the basis of the actual therapeutic program. On the other hand, in a case where the second mode is set by the mode setting unit 13, the therapeutic function execution unit 11 executes various processes on the basis of the pseudo-therapeutic program.

Timing at which the mode setting unit 13 sets any one mode with respect to the therapeutic application is, for example, timing before the subject actually uses the therapeutic application. For example, in a case where the subject activates the therapeutic application at timing before mode setting is not completed, a message indicating that mode setting is not completed is displayed, and the therapeutic application (function of the actual therapeutic program or the pseudo-therapeutic program) is locked not to be used. Then, when mode setting is executed by the mode setting unit 13, locking of the therapeutic application is released. According to this, in a case where the subject activates the therapeutic application at timing after the mode setting is completed, it enters a state in which the therapeutic application can be used.

Fig. 4 is a flowchart illustrating an operation example of the clinical trial system according to the first embodiment configured as described above. Note that, it is assumed that a doctor determines that a patient is to be registered as a clinical trial target subject through diagnosis on the patient, and the doctor inputs the subject specifying information and the assignment factor information in the doctor terminal 200 before executing the flowchart illustrated in Fig. 4. The flowchart illustrated in Fig. 4 is initiated when the subject specifying information and the assignment factor information input to the doctor terminal 200 are going to register in the clinical trial management server 300.

First, the doctor terminal 200 transmits the subject specifying information and the assignment factor information input by a doctor to the clinical trial management server 300 (step SI). In correspondence with this, the subject information input unit 21 inputs the subject specifying information and the assignment factor information (step S2), and stores these pieces of information in the assignment database storage unit 20A as a piece of information of the assignment database (step S3).

Here, details are not illustrated in the drawing for simplification of explanation, but subject specifying information and assignment factor information relating to a plurality of subjects are transmitted from a plurality of the doctor terminals 200 to the clinical trial management server 300, and pieces of information corresponding to the number of subjects necessary for the clinical trial are registered in the assignment database storage unit 20A as an assignment database.

In parallel with registration of the subject specifying information and the assignment factor information in the assignment database, the subject installs the therapeutic application in the subject terminal 100 (step S4). Note that, here, installation of the therapeutic application is illustrated in the drawing as a process in step S4 for convenience of explanation. However, after a patient is determined to be registered as the clinical trial subject through diagnosis by a doctor, installation of the therapeutic application may be executed at timing before registration of the subject specifying information and the assignment factor information in the assignment database.

Alternatively, in a case where the therapeutic application receives the assignment information from the clinical trial management server 300 in pull type communication, installation of the therapeutic application may be executed at arbitrary timing after registration of the subject specifying information and the assignment factor information in the assignment database. The reason for this is as follows. In the case of pull type communication, if a request for the assignment information is not made with the installed therapeutic application, the assignment information is not transmitted from the clinical trial management server 300.

Next, the random assignment unit 22 randomly assigns the subject to either the therapeutic group or the control group so that a target distribution is in an appropriate distributed state on the basis of the assignment factor information of the subject which is registered in the assignment database storage unit 20A as a part of the assignment database (step S5). Then, the random assignment unit 22 stores the assignment information indicating the assignment result in the assignment database storage unit 20A in association with the subject specifying information in the assignment database (step S6).

Next, the assignment information transmission unit 23 transmits the assignment information registered in the assignment database to the subject terminal 100 in the push type or the pull type (step S7). The assignment information reception unit 12 of the subject terminal 100 receives the assignment information transmitted from the clinical trial management server 300 (step S8).

The mode setting unit 13 sets the therapeutic application to either the first mode that provides the actual therapeutic function or the second mode that provides the pseudo-therapeutic function on the basis of the assignment information received by the assignment information reception unit 12 (step S9). After that, the therapeutic function execution unit 11 executes various processes on the basis of either the actual therapeutic program or the pseudo-therapeutic program in accordance with any one mode set by the mode setting unit 13.

As described above in detail, according to the first embodiment, in the clinical trial management server 300, assignment of the subject to the therapeutic group or the control group is randomly performed on the basis of the assignment factor information of the subject, and thus randomization of assignment to the therapeutic group and the control group is realized.

The assignment information indicating the assignment result is not disclosed to any of the subject terminal 100 and the doctor terminal 200, and is implicitly transmitted from the clinical trial management server 300 to the subject terminal 100. In addition, the therapeutic application installed in the subject terminal 100 is automatically set to either the first mode that provides the actual therapeutic function or the second mode that provides the pseudo-therapeutic function on the basis of the assignment information transmitted. According to this, therapy using the therapeutic application can be performed in a state in which both the subject and the doctor cannot understand that the subject is assigned to either the therapeutic group or the control group, and that the therapeutic application operates in which mode.

As described above, according to the clinical trial system according to the first embodiment, in the case of performing a clinical trial that applies the placebo-controlled test with respect to the therapeutic application, randomization and double-blindness of assignment to the therapeutic group and the control group can be systemically realized.

### (Second Embodiment)

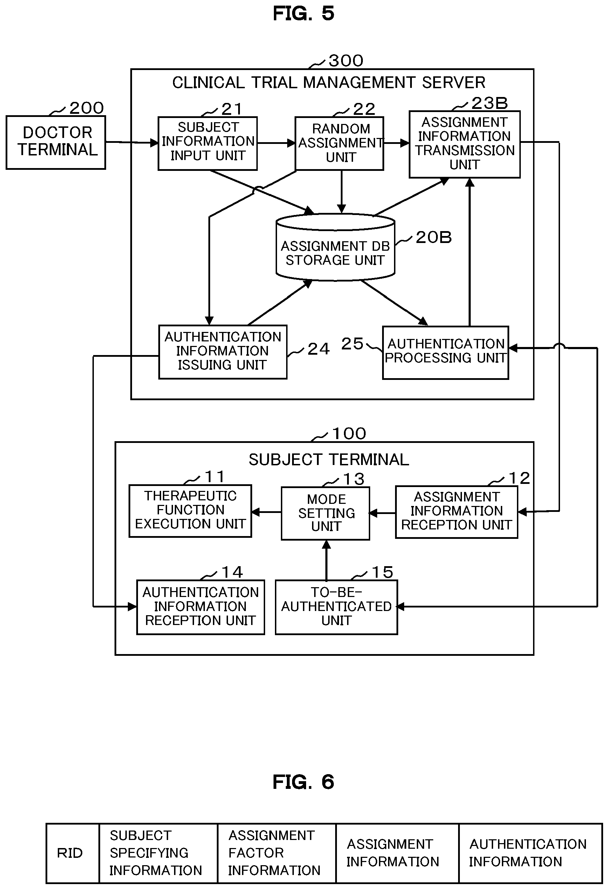

Next, a second embodiment of the invention will be described with reference to the accompanying drawings. A network configuration of a clinical trial system for a therapeutic application according to the second embodiment is similar to Fig. 1. Fig. 5 is a block diagram illustrating a functional configuration example of a subject terminal 100 and a clinical trial management server 300 according to the second embodiment. Note that, in Fig. 5, an element to which the same reference numeral as the reference numeral illustrated in Fig. 2 is given has the same function, and thus redundant description will be omitted here.

As illustrated in Fig. 5, the subject terminal 100 according to the second embodiment further includes an authentication information reception unit 14 and a to-be-authenticated unit 15 as a functional configuration. The functions of the authentication information reception unit 14 and the to-be-authenticated unit 15 are also realized through execution of the therapeutic application installed in the subject terminal 100.

In addition, the clinical trial management server 300 according to the second embodiment includes an assignment information transmission unit 23B instead of the assignment information transmission unit 23, and further includes an authentication information issuing unit 24 and an authentication processing unit 25 as a functional configuration. The functions of the assignment information transmission unit 23B, the authentication information issuing unit 24, and the authentication processing unit 25 can be constituted by any of hardware, a DSP, and software. In addition, the clinical trial management server 300 according to the second embodiment includes an assignment database storage unit 20B instead of the assignment database storage unit 20A as a storage medium.

The authentication information issuing unit 24 issues authentication information to be input when a subject uses the therapeutic application. Examples of the authentication information include a user ID and passwords. The authentication information issued by the authentication information issuing unit 24 is stored in the assignment database storage unit 20B as a part of the assignment database in association with the subject specifying information. Fig. 6 is a diagram illustrating an example of one record of the assignment database stored in the assignment database storage unit 20B. As illustrated in Fig. 6, the one record of the assignment database includes a record ID (RID), the subject specifying information, the assignment factor information, the assignment information, and the authentication information.

For example, timing at which the authentication information issuing unit 24 issues the authentication information can be set to timing at which assignment to the therapeutic group or the control group is performed with respect to a subject by the random assignment unit 22. In this case, in a stage in which the subject information input unit 21 inputs the subject specifying information and the assignment factor information, the pieces of information are recorded in the record, in a stage in which assignment by the random assignment unit 22 is performed, the assignment information is additionally recorded in the record, and in a stage in which the authentication information is issued by the authentication information issuing unit 24, the authentication information is additionally recorded in the record. Note that, the pieces of information noted here are pieces of minimum information necessary for describing the second embodiment, and may include other pieces of information.

The authentication information issued by the authentication information issuing unit 24 is registered in the assignment database storage unit 20B and is also transmitted to the subject terminal 100. Transmission of the authentication information may be performed by push type communication from the clinical trial management server 300 as in the transmission of the assignment information by the assignment information transmission unit 23 as described in the first embodiment, or may be performed by pull type communication in response to a request from the subject terminal 100. In addition, the authentication information issuing unit 24 may transmit the authentication information to the subject terminal 100 through the doctor terminal 200.

In the case of transmitting the authentication information in the push type, the authentication information issuing unit 24 issues the authentication information after being notified of assignment completion from the random assignment unit 22, stores the issued authentication information in the assignment database storage unit 20B, and transmits the authentication information to the subject terminal 100. On the other hand, in the case of transmitting the authentication information in the pull type, the authentication information issuing unit 24 stores the authentication information issued in response to the notification from the random assignment unit 22 in the assignment database storage unit 20B and waits, and reads out the authentication information corresponding to the subject specifying information from the assignment database storage unit 20B and transmits the authentication information to the subject terminal 100 in response to an authentication information issuing request including the subject specifying information from the subject terminal 100.

Note that, timing at which the authentication information issuing unit 24 issues the authentication information is not limited to the timing at which assignment with respect to the subject is performed by the random assignment unit 22. For example, the timing may be set to timing at which the subject information input unit 21 inputs the subject specifying information. In addition, after the subject installs the therapeutic application in the subject terminal 100, when the subject accesses the clinical trial management server 300 from the therapeutic application to make a request for the authentication information, the authentication information issuing unit 24 may issue the authentication information as a response to the request.

As to be described below, in the second embodiment, when authentication is successful, the assignment information is transmitted from the clinical trial management server 300 to the subject terminal 100. According to this, when the subject inputs the authentication information to the subject terminal 100 to be authenticated by the clinical trial management server 300, it is necessary for assignment by the random assignment unit 22 to be already completed. Accordingly, in the case of issuing the authentication information at the timing other than timing at which assignment is completed, it is preferable that the authentication information issuing unit 24 waits notification of assignment completion from the random assignment unit 22, and transmits the authentication information to the subject terminal 100 from the clinical trial management server 300.

The authentication information reception unit 14 of the subject terminal 100 receives the authentication information issued by the authentication information issuing unit 24. The authentication information is information that can be confirmed on a display of the subject terminal 100. A subject may record the authentication information confirmed on the display in a memo or stores the authentication information in a storage medium of the subject terminal 100 so that the authentication information can be used any time without forgetting.

The to-be-authenticated unit 15 receives authentication of the clinical trial management server 300 by using the authentication information issued by the authentication information issuing unit 24 (authentication information transmitted from the clinical trial management server 300). That is, the subject inputs the authentication information transmitted from the clinical trial management server 300 to the subject terminal 100. The to-be-authenticated unit 15 transmits the authentication information input to the subject terminal 100 by the subject to the clinical trial management server 300, and receives a result of authentication success or failure from the clinical trial management server 300.

Timing at which the to-be-authenticated unit 15 receives authentication of the clinical trial management server 300 is timing at which the therapeutic application installed in the subject terminal 100 is activated. Specifically, when a subject activates the therapeutic application, a log-in screen is displayed. In addition, when the subject inputs the authentication information including the user ID and the passwords to the log-in screen, the authentication information is transmitted to the clinical trial management server 300, and authentication is executed.

The authentication processing unit 25 of the clinical trial management server 300 authenticates validity of the input information transmitted from the subject terminal 100 by using the authentication information issued by the authentication information issuing unit 24 (authentication information stored in the assignment database storage unit 20B). Here, in a case where the authentication information stored in the assignment database storage unit 20B and the input information transmitted from the subject terminal 100 do not match each other, the authentication processing unit 25 returns the result indicating authentication failure to the to-be-authenticated unit 15. On the other hand, in a case where the authentication information stored in the assignment database storage unit 20B and the input information transmitted from the subject terminal 100 match each other, the authentication processing unit 25 returns the result indicating authentication success to the to-be-authenticated unit 15, and notifies the assignment information transmission unit 23B of the result.

The assignment information transmission unit 23B transmits the assignment information to the subject terminal 100 for which authentication by the authentication processing unit 25 is performed (which succeeds in authentication). In this embodiment, the assignment information transmission unit 23B transmits the assignment information to the subject terminal 100 in a push type only when the therapeutic application is installed in the subject terminal 100 and authentication is performed with respect to the subject terminal 100 for the first time by the authentication processing unit 25 (note that, since the assignment information is transmitted in response to authentication performed through log-in, this is referred to as one kind of pull type communication). With regard to transmission of the assignment information, when the authentication processing unit 25 transmits a message indicating authentication success to the subject terminal 100, the assignment information may be transmitted in combination with the message.

The mode setting unit 13 sets the therapeutic application to either the first mode or the second mode on the basis of the assignment information transmitted from the clinical trial management server 300 after first authentication by the to-be-authenticated unit 15. According to this, when a subject starts to actually use the therapeutic application, the therapeutic application is set to either the first mode or the second mode, and the therapeutic application can be caused to operate on the basis of either the actual therapeutic program or the pseudo-therapeutic program from the beginning.

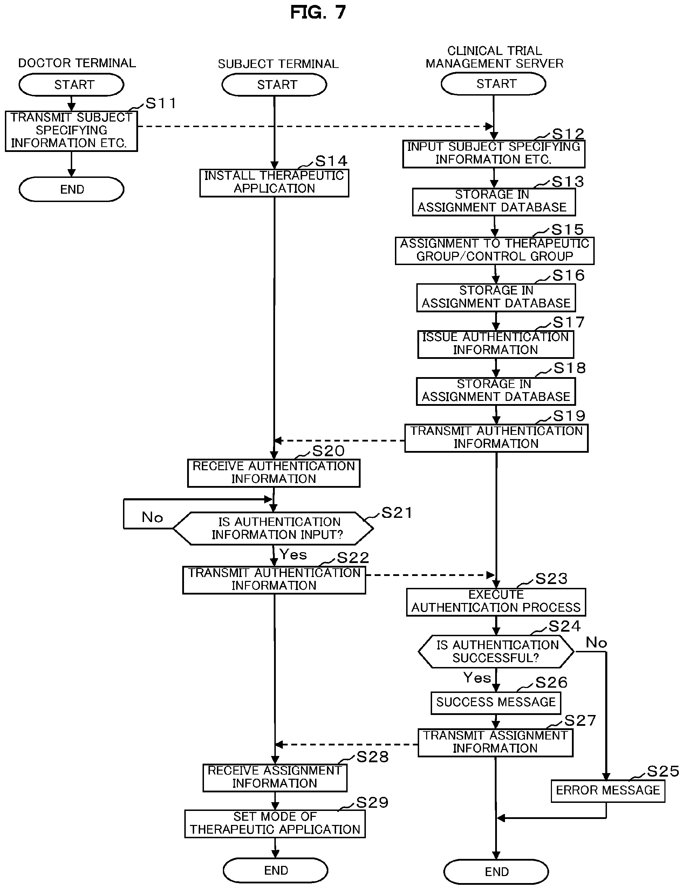

Fig. 7 is a flowchart illustrating an operation example of the clinical trial system according to the second embodiment configured as described above. Note that, it is assumed that a doctor determines that a patient is to be registered as a clinical trial target subject through diagnosis on the patient, and the doctor inputs the subject specifying information and the assignment factor information in the doctor terminal 200 before executing the flowchart illustrated in Fig. 7. The flowchart illustrated in Fig. 7 is initiated when the subject specifying information and the assignment factor information input to the doctor terminal 200 are going to register in the clinical trial management server 300.

First, the doctor terminal 200 transmits the subject specifying information and the assignment factor information input by a doctor to the clinical trial management server 300 (step S11). In correspondence with this, the subject information input unit 21 inputs the subject specifying information and the assignment factor information (step S12), and stores these pieces of information in the assignment database storage unit 20B as a piece of information of the assignment database (step S13).

Note that, here, details are not illustrated in the drawing for simplification of explanation, but subject specifying information and assignment factor information relating to a plurality of subjects are transmitted from a plurality of the doctor terminals 200 to the clinical trial management server 300, and pieces of information corresponding to the number of subjects necessary for the clinical trial are registered in the assignment database storage unit 20B as an assignment database.

In parallel with registration of the subject specifying information and the assignment factor information in the assignment database, the subject installs the therapeutic application in the subject terminal 100 (step S14). Note that, as described in the first embodiment, installation of the therapeutic application may be executed at timing before or after registration of the subject specifying information and the assignment factor information in the assignment database.

Next, the random assignment unit 22 randomly assigns the subject to either the therapeutic group or the control group so that a target distribution is in an appropriate distributed state on the basis of the assignment factor information of the subject which is registered in the assignment database storage unit 20B as a part of the assignment database (step S15). Then, the random assignment unit 22 stores the assignment information indicating the assignment result in the assignment database storage unit 20B in association with the subject specifying information in the assignment database (step S16).

Next, the authentication information issuing unit 24 issues authentication information with respect to each of subjects registered in the assignment database (step S17). The authentication information issuing unit 24 stores the issued authentication information in the assignment database storage unit 20B in association with the subject specifying information of the assignment database (step S18). In addition, the authentication information issuing unit 24 transmits the issued authentication information to the subject terminal 100 of subject (step S19). The authentication information reception unit 14 of the subject terminal 100 receives the authentication information issued by the authentication information issuing unit 24 (step S20).

The authentication information reception unit 14 of the subject terminal 100 receives the authentication information issued by the authentication information issuing unit 24 (step S31).

The flow up to this is a flow in which a process relating to the assignment information and the authentication information of the respective subjects is collectively performed in one process in a stage in which the subject specifying information and the assignment factor information of respective subjects are sequentially input and pieces of information corresponding to the number of subjects necessary for the clinical trial are gathered. In contrast, the following process is individually performed for every individual subject.

The to-be-authenticated unit 15 determines whether or not the authentication information is input to the subject terminal 100 by the subject (step S21). That is, the to-be-authenticated unit 15 determines whether or not the therapeutic application installed in step S14 is activated and the user ID and the passwords are input with respect to the log-in screen that is displayed at the time of the activation.

Here, in a case where the authentication information is not input, determination in step S21 continues. On the other hand, in a case where the authentication information is input to the subject terminal 100 by a user, the to-be-authenticated unit 15 transmits the input authentication information to the clinical trial management server 300 (step S22). In correspondence with the transmission, the authentication processing unit 25 of the clinical trial management server 300 authenticates validity of the input information transmitted from the subject terminal 100 by using authentication information stored in the assignment database storage unit 20B (step S23).

Here, the authentication processing unit 25 determines whether or not authentication is successful (step S24). In a case where the authentication fails, the authentication processing unit 25 transmits an error message indicating authentication failure to the subject terminal 100 (step S25), and terminates the process of the clinical trial management server 300 illustrated in Fig. 7. On the other hand, when the authentication is successful, the authentication processing unit 25 transmits the result indicating authentication success to the subject terminal 100 (step S26), and notifies the assignment information transmission unit 23B of the result.

In response to the notification, the assignment information transmission unit 23B transmits the assignment information registered in the assignment database to the subject terminal 100 in a push type (step S27). The assignment information reception unit 12 of the subject terminal 100 receives the assignment information transmitted from the clinical trial management server 300 (step S28).

The mode setting unit 13 sets the therapeutic application to either the first mode that provides the actual therapeutic function or the second mode that provides the pseudo-therapeutic function on the basis of the assignment information received by the assignment information reception unit 12 (step S29). After that, the therapeutic function execution unit 11 performs various processes on the basis of either the actual therapeutic program or the pseudo-therapeutic program in accordance with any one mode set by the mode setting unit 13.

According to the second embodiment configured as described above, in the case of performing the clinical trial that applies the placebo-controlled test with respect to the therapeutic application as in the first embodiment, the randomization and the double-blindness of assignment to the therapeutic group and the control group can be systemically realized.

In addition, according to the second embodiment, since mode setting with respect to the therapeutic application is performed at the first log-in, processes such as locking of the use of the therapeutic application until the mode setting is completed, and displaying of a message indicating activation of the therapeutic application at timing at which the mode setting is not completed are not necessary. According to this, convenience relating to initiation of the use of the therapeutic application by the subject can be improved.

In addition, in the second embodiment, since the authentication information issuing unit 24 transmits the authentication information to the subject terminal 100 after assignment by the random assignment unit 22, when authentication by the authentication processing unit 25 is successful, the assignment information can be reliably transmitted from the clinical trial management server 300 to the subject terminal 100. According to this, it is not necessary to perform a process of outputting an error message in a case where log-in is performed from the subject terminal 100 before assignment by the random assignment unit 22 is performed.

Note that, in the second embodiment, description has been given of an example in which the timing of the mode setting by the mode setting unit 13 is set to time of the first log-in with respect to the therapeutic application, but the invention is not limited thereto. For example, in the case where a function common to the therapeutic group and the control group is provided for a while after the subject initiates use of the therapeutic application, and the clinical trial is operated so that different functions are provided in the course of the clinical trial, for example, the mode setting for the therapeutic application may be performed at the time of second or later log-in. Alternatively, the mode setting may be performed at the time of log-in after passage of constant time from the time of the first log-in.

### (Third Embodiment)

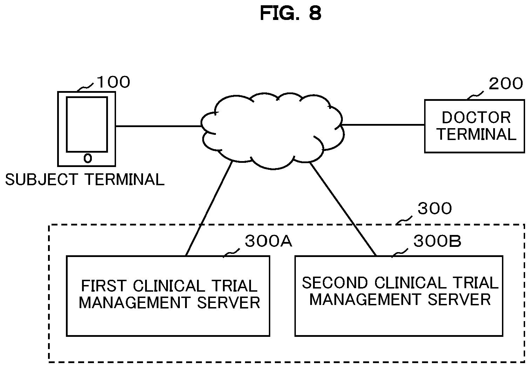

Next, a third embodiment of the invention will be described with reference to the accompanying drawings. Fig. 8 is a diagram illustrating a network configuration of a clinical trial system for a therapeutic application according to the third embodiment. As illustrated in Fig. 8, a clinical trial management server 300 according to the third embodiment includes a first clinical trial management server 300₋₁ and a second clinical trial management server 300₋₂.

A service provider that operates the first clinical trial management server 300₋₁ and a service provider that operates the second clinical trial management server 300₋₂ are different from each other. The first clinical trial management server 300₋₁ has a configuration similar to the subject information input unit 21, the random assignment unit 22, and the assignment information transmission unit 23B illustrated in Fig. 5, and executes a process of assigning a plurality of subjects to either the therapeutic group or the control group, and a process necessary for determining an operation mode of the therapeutic application. On the other hand, the second clinical trial management server 300₋₂ has a configuration similar to the authentication information issuing unit 24 and the authentication processing unit 25 illustrated in Fig. 5, and executes a process relating to authentication.

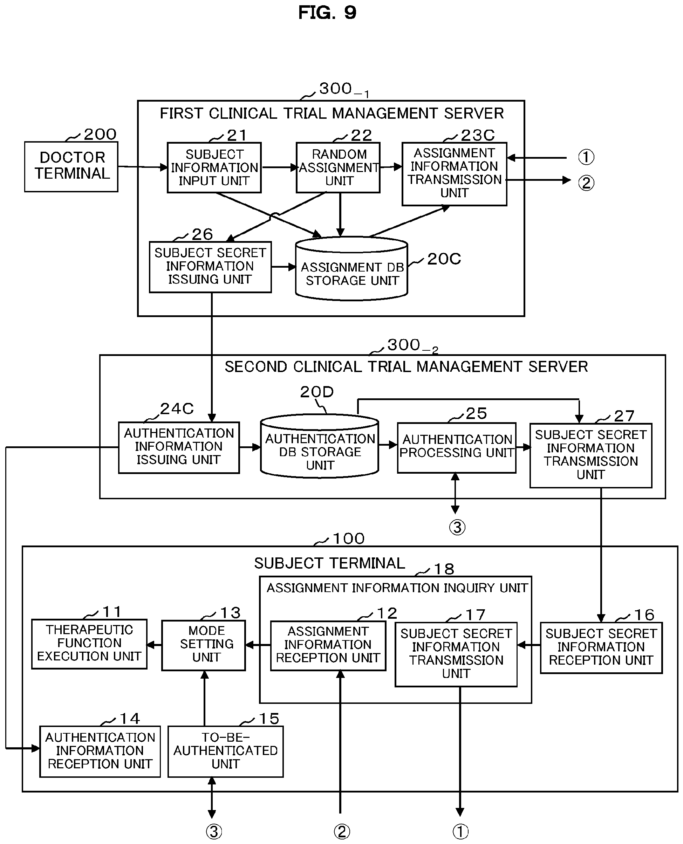

Fig. 9 is a block diagram illustrating a functional configuration example of the subject terminal 100 and the clinical trial management server 300 according to the third embodiment. Note that, in Fig. 9, an element to which the same reference numeral as the reference numeral illustrated in Fig. 5 is given has the same function, and thus redundant description will be omitted here.

As illustrated in Fig. 9, the subject terminal 100 according to the third embodiment further includes a subject secret information reception unit 16 and a subject secret information transmission unit 17 as a functional configuration. The subject secret information transmission unit 17 and the assignment information reception unit 12 constitute an assignment information inquiry unit 18. Functions of the subject secret information reception unit 16 and the subject secret information transmission unit 17 are realized through execution of the therapeutic application installed in the subject terminal 100.

The first clinical trial management server 300₋₁ according to the third embodiment includes a subject information input unit 21, a random assignment unit 22, an assignment information transmission unit 23C, and a subject secret information issuing unit 26 as a functional configuration. These functional blocks can be constituted by any of hardware, a DSP, and software. In addition, the first clinical trial management server 300₋₁ according to the third embodiment includes an assignment database storage unit 20C instead of the assignment database storage unit 20B as a storage medium.

The second clinical trial management server 300₋₂ according to the third embodiment includes an authentication information issuing unit 24C, an authentication processing unit 25, and a subject secret information transmission unit 27 as a functional configuration. These functional blocks can be constituted by any of hardware, a DSP, and software. In addition, the second clinical trial management server 300₋₂ according to the third embodiment includes an authentication database storage unit 20D as a storage medium.

The subject secret information issuing unit 26 provided in the first clinical trial management server 300₋₁ issues subject secret information associated with subject specifying information input by the subject information input unit 21. The subject secret information is information that cannot specify a subject by itself. For example, the subject secret information can be set to a subject number that is different from the subject specifying information, and cannot be guessed from the subject specifying information.

Timing at which the subject secret information issuing unit 26 issues the subject secret information may be arbitrary timing after the subject specifying information is input by the subject information input unit 21, but it is preferable that the timing is set to timing at which assignment to the therapeutic group or the control group with respect to the subject is performed by the random assignment unit 22.

The subject secret information issuing unit 26 stores the issued subject secret information in the assignment database storage unit 20C as a part of the assignment database in association with the subject specifying information. In addition, the subject secret information issuing unit 26 notifies the authentication information issuing unit 24C provided in the second clinical trial management server 300₋₂ of the issued subject secret information.

The authentication information issuing unit 24C receives the subject secret information from the first clinical trial management server 300₋₁ and issues the authentication information in association with the subject secret information. As described above, the subject secret information issuing unit 26 issues the subject secret information at timing at which assignment to the therapeutic group or the control group is performed with respect to the subject by the random assignment unit 22, and the authentication information issuing unit 24 receives the subject secret information and issues the authentication information. For this reason, the timing at which the authentication information is issued becomes timing at which assignment by the random assignment unit 22 is performed as in the second embodiment.

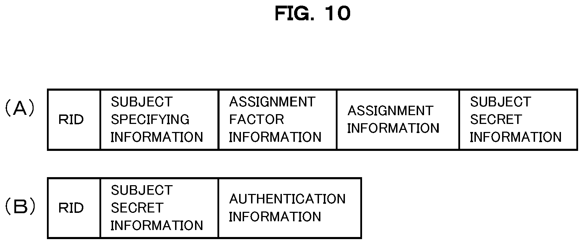

The authentication information issued by the authentication information issuing unit 24C is stored in the authentication database storage unit 20D as a part of the authentication database in association with the subject secret information. Fig. 10 is a diagram illustrating an example of one record of each of the assignment database stored in the assignment database storage unit 20C and the authentication database stored in the authentication database storage unit 20D.

As illustrated in Fig. 10(A), one record of the assignment database stored in the assignment database storage unit 20C includes a record ID (RID), the subject specifying information, the assignment factor information, the assignment information, and the subject secret information. That is, in a stage in which the subject information input unit 21 inputs the subject specifying information and the assignment factor information, these pieces of information are recorded in the record, in a stage in which assignment is performed by the random assignment unit 22, the assignment information is additionally recorded in the record, and in a stage in which the subject secret information is issued by the subject secret information issuing unit 26, the subject secret information is additionally recorded in the record. Note that, the pieces of information noted here are pieces of minimum information necessary for describing the third embodiment, and may include other pieces of information.

In addition, as illustrated in Fig. 10(B), one record of the authentication database stored in the authentication database storage unit 20D includes a record ID (RID), the subject secret information, and the authentication information. That is, in a stage in which authentication information is issued by the authentication information issuing unit 24C, the authentication information is recorded in a record of the authentication database storage unit 20D in combination with the subject secret information. Note that, the information noted here are pieces of minimum information necessary for describing the third embodiment, and may include other pieces of information.

The authentication information issued by the authentication information issuing unit 24C is transmitted to the subject terminal 100. Transmission of the authentication information can be performed through pull type communication in response to a request from the subject terminal 100, but it is preferable that the transmission is performed by push type communication from the clinical trial management server 300. In this case, the authentication information issuing unit 24C may indirectly transmit the authentication information to the subject terminal 100 through the doctor terminal 200.

Note that, for example, destination information of the subject terminal 100 which is used when the authentication information issuing unit 24C transmits the authentication information to the subject terminal 100 in the push type may be transmitted from the doctor terminal 200 to the first clinical trial management server 300₋₁ and registered in the assignment database as a piece of the subject specifying information, and the destination information may also be transmitted in combination when the subject secret information issuing unit 26 notifies the authentication information issuing unit 24 of the subject secret information.

The subject secret information transmission unit 27 provided in the second clinical trial management server 300₋₂ transmits the subject secret information stored in the authentication database storage unit 20D to the subject terminal 100 after validity of the input information transmitted from the subject terminal 100 is authenticated by the authentication processing unit 25. With regard to transmission of the subject secret information, when the authentication processing unit 25 transmits a message indicating authentication success to the subject terminal 100, the subject secret information may be transmitted in combination with the message.

The subject secret information reception unit 16 of the subject terminal 100 receives the subject secret information transmitted by the subject secret information transmission unit 27. The subject secret information is information which cannot specify a subject, and thus the subject secret information is information that can be published on a display of the subject terminal 100. The subject confirms the subject secret information displayed on the display, and inputs the subject secret information to the subject terminal 100.

The assignment information inquiry unit 18 transmits the subject secret information input to the subject terminal 100 to the first clinical trial management server 300₋₁ as described above, and acquires assignment information corresponding to the subject secret information. That is, the subject secret information transmission unit 17 that constitutes the assignment information inquiry unit 18 transmits the subject secret information to the first clinical trial management server 300₋₁. The assignment information transmission unit 23C of the first clinical trial management server 300₋₁ reads out the assignment information associated with the subject secret information received from the subject terminal 100 from the assignment database storage unit 20C, and transmits the assignment information to the subject terminal 100 that is a transmission source of the subject secret information. The assignment information reception unit 12 that constitutes the assignment information inquiry unit 18 receives the assignment information.

Note that, here, the subject secret information input to the subject terminal 100 by the subject is transmitted to the first clinical trial management server 300₋₁, but there is no limitation thereto. For example, the subject secret information transmission unit 17 may automatically transmit the subject secret information received from the second clinical trial management server 300₋₂ by the subject secret information reception unit 16 to the first clinical trial management server 300₋₁.

The mode setting unit 13 of the subject terminal 100 sets the therapeutic application to either the first mode or the second mode on the basis of the assignment information acquired by the assignment information inquiry unit 18.

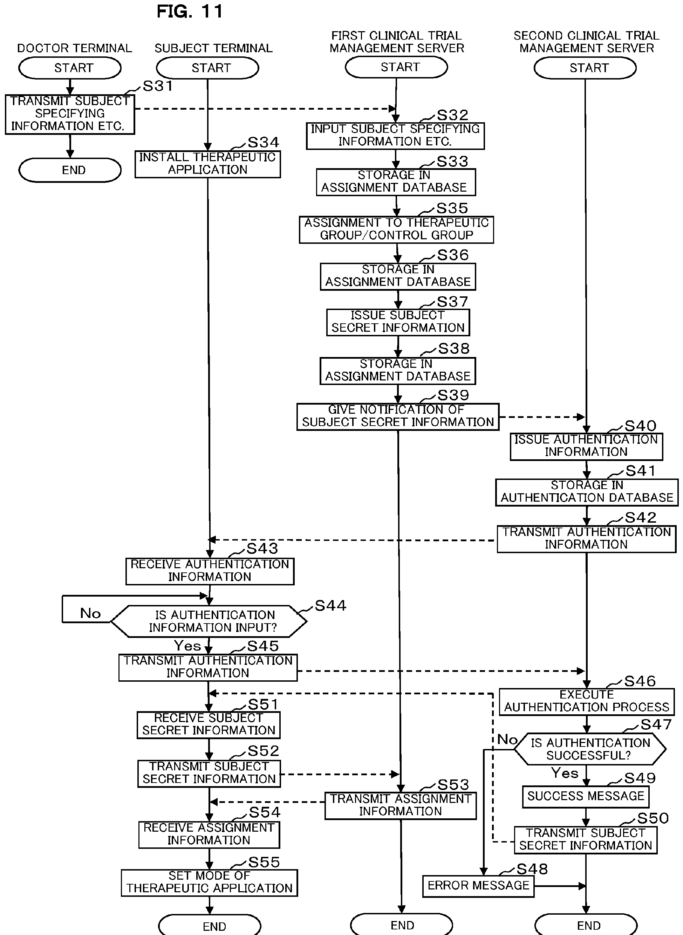

Fig. 11 is a flowchart illustrating an operation example of the clinical trial system according to the third embodiment configured as described above. Note that, it is assumed that a doctor determines that a patient is to be registered as a clinical trial target subject through diagnosis on the patient, and the doctor inputs the subject specifying information and the assignment factor information in the doctor terminal 200 before executing the flowchart illustrated in Fig. 11. The flowchart illustrated in Fig. 11 is initiated when the subject specifying information and the assignment factor information input to the doctor terminal 200 are going to register in the first clinical trial management server 300₋₁.

First, the doctor terminal 200 transmits the subject specifying information and the assignment factor information input by a doctor to the first clinical trial management server 300₋₁ (step S31). In correspondence with this, the subject information input unit 21 inputs the subject specifying information and the assignment factor information (step S32), and stores these pieces of information in the assignment database storage unit 20C as a piece of information of the assignment database (step S33).

Note that, here, details are not illustrated in the drawing for simplification of explanation, but subject specifying information and assignment factor information relating to a plurality of subjects are transmitted from a plurality of the doctor terminals 200 to the first clinical trial management server 300₋₁, and pieces of information corresponding to the number of subjects necessary for the clinical trial are registered in the assignment database storage unit 20C as an assignment database.

In parallel with registration of the subject specifying information and the assignment factor information in the assignment database, the subject installs the therapeutic application in the subject terminal 100 (step S34). Note that, as described in the first embodiment, installation of the therapeutic application may be executed at timing before or after registration of the subject specifying information and the assignment factor information in the assignment database.

Next, the random assignment unit 22 randomly assigns the subject to either the therapeutic group or the control group so that a target distribution is in an appropriate distributed state on the basis of the assignment factor information of the subject which is registered in the assignment database storage unit 20C as a part of the assignment database (step S35). In addition, the random assignment unit 22 stores the assignment information indicating the assignment result in the assignment database storage unit 20C in association with the subject specifying information in the assignment database (step S36).

Next, the subject secret information issuing unit 26 issues subject secret information associated with the subject specifying information input by the subject information input unit 21 in step S32 (step S37). In addition, the subject secret information issuing unit 26 stores the issued subject secret information in the assignment database storage unit 20C in association with the subject specifying information in the assignment database (step S38). In addition, the subject secret information issuing unit 26 notifies the authentication information issuing unit 24C of the second clinical trial management server 300₋₂ of the issued subject secret information (step S39).

In response to the notification, the authentication information issuing unit 24C issues authentication information with respect to each pieces of subject secret information given in notification (step S40). In addition, the authentication information issuing unit 24C stores the issued authentication information in the authentication database storage unit 20D in combination with the subject secret information (step S41). In addition, the authentication information issuing unit 24C transmits the issued authentication information to the subject terminal 100 of each of subjects (step S42). The authentication information reception unit 14 of the subject terminal 100 receives the authentication information issued by the authentication information issuing unit 24C (step S43).

The flow up to this is a flow in which a process relating to the assignment information and the authentication information of the respective subjects is collectively performed in one process in a stage in which the subject specifying information and the assignment factor information of respective subjects are sequentially input and pieces of information corresponding to the number of subjects necessary for the clinical trial are gathered. In contrast, the following process is individually performed for every individual subject.

The to-be-authenticated unit 15 determines whether or not the authentication information is input to the subject terminal 100 by the subject (step S44). That is, the to-be-authenticated unit 15 determines whether or not the therapeutic application installed in step S34 is activated and the user ID and the passwords are input with respect to the log-in screen that is displayed at the time of the activation.

Here, in a case where the authentication information is not input, determination in step S44 continues. On the other hand, in a case where the authentication information is input to the subject terminal 100 by a user, the to-be-authenticated unit 15 transmits the input authentication information to the second clinical trial management server 300₋₂ (step S45). In correspondence with the transmission, the authentication processing unit 25 of the second clinical trial management server 300₋₂ authenticates validity of the input information transmitted from the subject terminal 100 by using authentication information stored in the authentication database storage unit 20D (step S46).

Here, the authentication processing unit 25 determines whether or not authentication is successful (step S47). In a case where the authentication fails, the authentication processing unit 25 transmits an error message indicating authentication failure to the subject terminal 100 (step S48), and terminates the process of the second clinical trial management server 300₋₂ illustrated in Fig. 11. On the other hand, when the authentication is successful, the authentication processing unit 25 transmits a message indicating authentication success to the subject terminal 100 (step S49), and notifies the subject secret information transmission unit 27 of the result.

In response to the notification, the subject secret information transmission unit 27 transmits the subject secret information registered in the assignment database to the subject terminal 100 in a push type (step S50). The subject secret information reception unit 16 of the subject terminal 100 receives the subject secret information transmitted from the clinical trial management server 300 (step S51).

When the subject secret information reception unit 16 receives the subject secret information, the subject secret information transmission unit 17 transmits the subject secret information to the first clinical trial management server 300₋₁ to make a request for the assignment information (step S52). In response to the request, the assignment information transmission unit 23C transmits the assignment information registered in the assignment database in association with the subject secret information to the subject terminal 100 (step S53). In correspondence with this, the assignment information reception unit 12 of the subject terminal 100 receives the assignment information transmitted from the clinical trial management server 300 (step S54).

The mode setting unit 13 sets the therapeutic application to either the first mode or the second mode on the basis of the assignment information received by the assignment information reception unit 12 (step S55). After that, the therapeutic function execution unit 11 performs various processes on the basis of either the actual therapeutic program or the pseudo-therapeutic program in accordance with any one mode set by the mode setting unit 13.

According to the third embodiment configured as described above, in the case of performing the clinical trial that applies the placebo-controlled test with respect to the therapeutic application as in the first embodiment, the randomization and the double-blindness of assignment to the therapeutic group and the control group can be systemically realized.

In addition, as in the second embodiment, processes such as locking of the use of the therapeutic application until the mode setting is completed, displaying of a message indicating activation of the therapeutic application at timing at which the mode setting is not completed, and displaying of an error message in a case where log-in is performed before assignment with respect to a subject are not necessary. According to this, convenience relating to initiation of the use of the therapeutic application by the subject can be improved.

In addition, in the third embodiment, the clinical trial management server 300 are divided into the first clinical trial management server 300₋₁ and the second clinical trial management server 300₋₂, a process relating to assignment of a subject and mode setting of the therapeutic application, and a process relating to authentication of the subject are carried out by service providers different from each other. In addition, communication between the subject terminal 100, the first clinical trial management server 300₋₁, and second clinical trial management server 300₋₂ is carried out by using the subject secret information that cannot specify the subject. According to this, it can realized the situation in manner that not only the doctor and the subject, but also the service provider who operates the clinical trial are completely unaware of that the subject is assigned to either the therapeutic group or the control group.

Note that, in the third embodiment, description has been given of a configuration example in which the clinical trial management server 300 is divided into the first clinical trial management server 300₋₁ and the second clinical trial management server 300₋₂ on the basis of the second embodiment. In contrast, the clinical trial management server 300 may be divided into the first clinical trial management server 300₋₁ and the second clinical trial management server 300₋₂ on the basis of the first embodiment.

In addition, in the first to third embodiment, description has been given of an example in which the process of assigning each subject is performed by the random assignment unit 22 in a stage in which the subject specifying information and the assignment factor information of respective subjects are sequentially input from the plurality of doctor terminals 200 to the clinical trial management server 300 and pieces of information corresponding to the number of subjects necessary for the clinical trial are registered in the assignment database, but the invention is not limited thereto. For example, the process of assigning the subject may be performed whenever the subject specifying information and the assignment factor information of one subject are input. In this case, when performing assignment with respect to the one subject, the random assignment unit 22 may randomly assign the subject to either the therapeutic group or the control group so that a target distribution is in an appropriate distributed state with reference to the assignment factor information and the assignment information relating to the subject which are registered in the assignment database.

In addition, each of the first to third embodiments merely illustrates a specific example for carrying out the invention, and it should be understood that the technical scope of the invention is not limitedly interpreted by the embodiments. That is, the invention can be implemented in various forms without departing form the gist or main features of the invention.

### Reference Signs List

- 11: Therapeutic function execution unit
- 12: Assignment information reception unit
- 13: Mode setting unit
- 14: Authentication information reception unit
- 15: To-be-authenticated unit
- 16: Subject secret information reception unit
- 17: Subject secret information transmission unit
- 18: Assignment information inquiry unit
- 20A, 20B, 20C: Assignment database storage unit
- 20D: Authentication database storage unit
- 21: Subject information input unit
- 22: Random assignment unit
- 23, 23B, 23C: Assignment information transmission unit
- 24, 24C: Authentication information issuing unit
- 25: Authentication processing unit
- 26: Subject secret information issuing unit
- 27: Subject secret information transmission unit
- 100: Subject terminal
- 200: Doctor terminal
- 300: Clinical trial management server
- 300₋₁: First clinical trial management server
- 300₋₂: Second clinical trial management server

## Claims

1. A clinical trial system for a therapeutic application, comprising a subject terminal with which a subject uses a therapeutic application; a doctor terminal that is used by a doctor; and a clinical trial management server that performs management of a clinical trial; the subject terminal, the doctor terminal and the clinical trial management server conducting the clinical trial of the therapeutic application in cooperation, **characterized in that**
the clinical trial management server includes,
a subject information input unit that inputs subject specifying information capable of specifying the subject, and assignment factor information to be referred to when assigning the subject to either a therapeutic group or a control group from the doctor terminal,
a random assignment unit that randomly assigns the subject to either the therapeutic group or the control group so that a target distribution is in an appropriate distributed state on the basis of the assignment factor information input by the subject information input unit, and
an assignment information transmission unit that directly or indirectly transmits assignment information indicating an assignment result to either the therapeutic group or the control group by the random assignment unit to the subject terminal, and
the subject terminal includes,
a mode setting unit that sets the therapeutic application to either a first mode that provides an actual therapeutic function or a second mode that provides a pseudo-therapeutic function on the basis of the assignment information transmitted from the clinical trial management server.

2. The clinical trial system for a therapeutic application according to claim 1, **characterized in that**
the clinical trial management server further includes,
an authentication information issuing unit that issues authentication information to be input when the subject uses the therapeutic application, and
an authentication processing unit that authenticates validity of input information transmitted from the subject terminal by using the authentication information issued by the authentication information issuing unit,
the assignment information transmission unit transmits the assignment information with respect to the subject terminal for which authentication by the authentication processing unit is performed,
the subject terminal further includes,
a to-be-authenticated unit that receives authentication of the clinical trial management server by using the authentication information issued by the authentication information issuing unit, and
the mode setting unit sets the therapeutic application to either the first mode or the second mode on the basis of the assignment information transmitted from the clinical trial management server after authentication by the to-be-authenticated unit.

3. The clinical trial system for a therapeutic application according to claim 2, **characterized in that**
the mode setting unit sets the therapeutic application to either the first mode or the second mode on the basis of the assignment information transmitted from the clinical trial management server after first authentication by the to-be-authenticated unit.

4. The clinical trial system for a therapeutic application according to claim 2 or 3, **characterized in that**
the clinical trial management server includes,
a first clinical trial management server including the subject information input unit, the random assignment unit, and the assignment information transmission unit, and
a second clinical trial management server including the authentication information issuing unit and the authentication processing unit.

5. The clinical trial system for a therapeutic application according to claim 4, **characterized in that**
the first clinical trial management server further includes a subject secret information issuing unit that issues subject secret information associated with the subject specifying information, and
the authentication information issuing unit of the second clinical trial management server receives the subject secret information from the first clinical trial management server, and issues the authentication information in association with the subject secret information.

6. The clinical trial system for a therapeutic application according to claim 5, **characterized in that**
the second clinical trial management server further includes a subject secret information transmission unit that transmits the subject secret information to the subject terminal after validity of input information transmitted from the subject terminal is authenticated by the authentication processing unit,
the subject terminal further includes an assignment information inquiry unit that transmits the subject secret information received from the second clinical trial management server to the first clinical trial management server, and acquires assignment information corresponding to the subject secret information,
the assignment information transmission unit of the clinical trial management server transmits the assignment information with respect to the subject terminal that is a transmission source of the subject secret information, and
the mode setting unit of the subject terminal sets the therapeutic application to either the first mode or the second mode on the basis of the assignment information acquired by the assignment information inquiry unit.

7. A terminal device that is equipped with a therapeutic application used by a subject, and cooperates with a clinical trial management server that conducts a clinical trial of the therapeutic application, the terminal device **characterized by** comprising:
a to-be-authenticated unit that receives authentication of the clinical trial management server by using authentication information issued by the clinical trial management server; and
a mode setting unit that sets the therapeutic application to either a first mode that provides an actual therapeutic function or a second mode that provides a pseudo-therapeutic function on the basis of assignment information that is transmitted from the clinical trial management server after authentication by the to-be-authenticated unit, and that indicates a result of assignment to either a therapeutic group or a control group on the basis of assignment factor information of the subject.

8. The terminal device that is equipped with a therapeutic application according to claim 7, further comprising:
a subject specifying information reception unit that receives subject secret information associated with subject specifying information capable of specifying the subject from a second clinical trial management server when the to-be-authenticated unit receives authentication by the second clinical trial management server that is one of the clinical trial management server; and
an assignment information inquiry unit that transmits the subject secret information received by the subject specifying information reception unit to a first clinical trial management server that is one of the clinical trial management server, and acquires assignment information corresponding to the subject secret information,
wherein the mode setting unit sets the therapeutic application to either the first mode or the second mode on the basis of the assignment information acquired by the assignment information inquiry unit.

9. A therapeutic application program that has a first mode providing actual therapeutic function and a second mode providing pseudo-therapeutic function and operates in accordance with any one mode that is selectively set, the therapeutic application program causing a computer to function as:
a to-be-authenticated means that receives authentication of the clinical trial management server by using authentication information issued by the clinical trial management server that performs management of a clinical trial of the therapeutic application; and
a mode setting means that sets the therapeutic application to either the first mode or the second mode on the basis of assignment information that is transmitted from the clinical trial management server after authentication by the to-be-authenticated means, and that indicates a result of assignment to either a therapeutic group or a control group on the basis of assignment factor information of the subject.

10. The therapeutic application program according to claim 9, further causing the computer to function as:
a subject specifying information reception means that receives subject secret information associated with subject specifying information capable of specifying the subject from a second clinical trial management server when the to-be-authenticated means receives authentication by the second clinical trial management server that is one of the clinical trial management server; and
an assignment information inquiry means that transmits the subject secret information received by the subject specifying information reception means to a first clinical trial management server that is one of the clinical trial management server, and acquires assignment information corresponding to the subject secret information, **characterized in that**
the mode setting means sets the therapeutic application to either the first mode or the second mode on the basis of the assignment information acquired by the assignment information inquiry means.
